# EUROPÄISCHE PATENTANMELDUNG

(11) **EP 3 424 301 A1**
(43) Veröffentlichungstag der Anmeldung: **09.01.2019**
(21) Anmeldenummer: 17179675.8
(22) Anmeldetag: 04.07.2017
(51) Int. Cl.: A01G 20/47

(54) **VERFAHREN ZUM ERMITTELN EINES VERSCHMUTZUNGSZUSTANDS MINDESTENS EINER KOMPONENTE EINES GARTEN- UND/ODER FORSTGERÄTS UND GARTEN- UND/ODER FORSTGERÄTESYSTEM**

(71) Anmelder: Andreas Stihl AG & Co. KG, 71336 Waiblingen (DE)
(72) Erfinder: Frey, Benjamin, 73663 Berglen (DE); Reinert, Michael, 73635 Rudersberg (DE); Hollmeier, Friedrich, 73635 Rudersberg (DE); Eppinger, Andreas, 70599 Stuttgart (DE); Fernandez Krämer, Antonio, 70180 Stuttgart (DE)
(74) Vertreter: Patentanwälte Ruff, Wilhelm, Beier, Dauster & Partner mbB

(57) **Zusammenfassung**

Die Erfindung bezieht sich auf ein Verfahren zum Ermitteln eines Verschmutzungszustands (VZ) mindestens einer Komponente (30) eines Garten- und/oder Forstgeräts (20) und ein Garten- und/oder Forstgerätesystem (10).

## Beschreibung

### ANWENDUNGSGEBIET UND STAND DER TECHNIK

Die Erfindung bezieht sich auf ein Verfahren zum Ermitteln eines Verschmutzungszustands mindestens einer Komponente eines Garten- und/oder Forstgeräts und ein Garten- und/oder Forstgerätesystem.

Ein Verfahren zum Ermitteln eines Verschmutzungszustands mindestens einer Komponente eines Garten- und/oder Forstgeräts und ein Garten- und/oder Forstgerätesystem sind bekannt.

### AUFGABE UND LÖSUNG

Der Erfindung liegt als Aufgabe die Bereitstellung eines Verfahrens zum Ermitteln eines Verschmutzungszustands mindestens einer Komponente eines Garten- und/oder Forstgeräts zugrunde, das verbesserte Eigenschaften aufweist, insbesondere mehr Funktionalitäten. Des Weiteren liegt der Erfindung als Aufgabe die Bereitstellung eines Garten- und/oder Forstgerätesystems zugrunde.

Die Erfindung löst diese Aufgabe durch die Bereitstellung eines Verfahrens mit den Merkmalen des Anspruchs 1. Des Weiteren wird die der Erfindung zugrundeliegende Aufgabe durch ein Garten- und/oder Forstgerätesystem mit den Merkmalen des Anspruchs 13 gelöst. Vorteilhafte Weiterbildungen und/oder Ausgestaltungen der Erfindung sind in den abhängigen Ansprüchen beschrieben.

Das erfindungsgemäße, insbesondere automatische, Verfahren zum Ermitteln eines, insbesondere angenommenen tatsächlichen oder voraussichtlichen, Verschmutzungszustands oder Verschmutzungsgrads mindestens einer Komponente eines Garten- und/oder Forstgeräts weist die Schritte auf: a) Ermitteln, insbesondere automatisches Ermitteln, mindestens eines Schmutzparameters, der mindestens eine während eines Betriebs des Garten- und/oder Forstgeräts vorherrschende Verschmutzungsursache beschreibt. b) Ermitteln, insbesondere automatisches Ermitteln, des Verschmutzungszustands der Komponente basierend auf dem ermittelten Schmutzparameter. c) Ausgeben, insbesondere automatisches Ausgeben, und/oder Übertragen, insbesondere automatisches Übertragen, einer auf dem ermittelten Verschmutzungszustand basierenden Information.

Das Verfahren ermöglicht ein mittelbares Ermitteln des Verschmutzungszustands der Komponente. Anders formuliert: der Verschmutzungszustand der Komponente braucht nicht unmittelbar ermittelt zu werden. Dies kann ermöglichen, den Verschmutzungszustand der Komponente bereits vor dem Betrieb des Garten- und/oder Forstgeräts zu ermitteln oder zu prognostizieren, insbesondere wenn die mindestens eine während des Betriebs des Garten- und/oder Forstgeräts vorherrschende Verschmutzungsursache bereits vor dem Betrieb bekannt sein kann oder ermittelt, insbesondere prognostiziert, werden kann.

Zusätzlich oder alternativ ermöglicht das Verfahren aufgrund der Information nach der Komponente zu schauen, insbesondere bevor diese zu stark verschmutzt sein kann. Insbesondere kann bei Bedarf rechtzeitig Reinigungs- und/oder Wartungsmaterial und/oder eine Ersatzkomponente besorgt und/oder mitgeführt werden, insbesondere durch einen Garten- und/oder Forstarbeiter beziehungsweise einen Benutzer des Garten- und/oder Forstgeräts. Somit ermöglicht das Verfahren, das Garten- und/oder Forstgerät beziehungsweise seine Komponente vor Schlechtleistung und/oder Beschädigung aufgrund von zu starker Verschmutzung der Komponente zu schützen.

Insbesondere kann die Information optisch, akustisch und/oder haptisch ausgegeben werden. Das optische Ausgeben kann ein Anzeigen aufweisen. Das optische Ausgeben kann ein Einblenden oder Projizieren der Information in ein Sichtfeld des Garten- und/oder Forstarbeiters aufweisen. Zusätzlich oder alternativ kann die Information an eine Datenbank und/oder ein Netzwerk und/oder einen Hub und/oder eine Cloud und/oder ähnliches übertragen werden, insbesondere an das Internet.

Das Garten- und/oder Forstgerät kann als ein handgeführtes Garten- und/oder Forstgerät ausgebildet sein, insbesondere als ein handgetragenes Garten- und/oder Forstgerät oder ein bodengeführtes Garten- und/oder Forstgerät. Handgeführtes, insbesondere handgetragenes, Garten- und/oder Forstgerät kann bedeuten, dass das Garten- und/oder Forstgerät eine maximale Masse von 50 Kilogramm (kg), insbesondere von 20 kg, insbesondere von 10 kg, aufweisen kann.

Der Schmutzparameter kann einen Wert und/oder einen Betrag aufweisen oder sein. Der Verschmutzungszustand kann einen Wert und/oder einen Betrag aufweisen oder sein.

Der Schritt b) kann zeitgleich mit dem Schritt a) und/oder zeitlich nach diesem ausgeführt werden. Der Schritt c) kann zeitgleich mit dem Schritt b) und/oder zeitlich nach diesem ausgeführt werden.

In einer Weiterbildung der Erfindung weist der Schritt a) auf: Ermitteln oder Erfassen des Schmutzparameters mittels mindestens eines Sensors, der getrennt von der Komponente, insbesondere von dem Garten- und/oder Forstgerät, ausgebildet ist. In anderen Worten: der Sensor kann oder braucht kein Bestandteil der Komponente sein, insbesondere kein Bestandteil des Garten- und/oder Forstgeräts. Insbesondere kann der Sensor dazu ausgebildet sein, die Verschmutzungsursache unmittelbar zu erfassen oder zu messen. Anders formuliert: der Sensor kann oder braucht nicht dazu ausgebildet sein, die Komponente zu messen, insbesondere zu vermessen.

In einer Weiterbildung der Erfindung ist der Verschmutzungszustand der Komponente von dem Betrieb des Garten- und/oder Forstgeräts abhängig. Die Komponente ist während des Betriebs des Garten- und/oder Forstgeräts der Verschmutzungsursache ausgesetzt. Das Verfahren weist den Schritt auf: Ermitteln, insbesondere automatisches Ermitteln, von Daten des Betriebs des Garten- und/oder Forstgeräts. Der Schritt b) weist auf: Ermitteln des Verschmutzungszustands der Komponente basierend auf den ermittelten Daten des Betriebs. Dies ermöglicht eine relativ genaue Ermittlung des Verschmutzungszustands. Der Schritt b) kann zeitgleich oder zeitlich nach dem Ermitteln der Daten des Betriebs ausgeführt werden.

In einer Ausgestaltung der Erfindung weisen die Daten des Betriebs eine Laufzeit des Garten- und/oder Forstgeräts auf, insbesondere einen Drehzahlverlauf, insbesondere über der Laufzeit, des Garten- und/oder Forstgeräts. Insbesondere kann die Laufzeit durch Erfassen eines von dem Betrieb des Garten- und/oder Forstgeräts erzeugten Schalls ermittelt werden, insbesondere mittels des zuvor beschriebenen Sensors. Der Sensor kann einen Schallwandler aufweisen, insbesondere ein Mikrophon. Die Laufzeit und insbesondere der Drehzahlverlauf können von einer Arbeitsweise mit dem Garten- und/oder Forstgerät abhängen oder zumindest beeinflusst sein. Entsprechend kann der Verschmutzungszustand von der Arbeitsweise abhängen oder zumindest beeinflusst sein. Die Laufzeit kann einen Wert und/oder einen Betrag aufweisen oder sein, insbesondere in Sekunden, Minuten, Stunden, Tagen und/oder einer größeren Zeiteinheit. Der Drehzahlverlauf kann einen Wert beziehungsweise eine Wertemenge und/oder einen Betrag aufweisen oder sein, insbesondere in der Einheit Umdrehungen pro Minute.

In einer Weiterbildung der Erfindung weist die Komponente einen Zylinder, ein Lüfterrad, ein Luftfiltergitter, einen Luftfilter und/oder eine Kühlrippe auf oder ist eine solche spezifische Komponente. In der Regel kann eine derart weitergebildete Komponente mit einer Umgebungsluft des Garten- und/oder Forstgeräts wechselwirken, insbesondere zur Verbrennung der Umgebungsluft und/oder zur Kühlung mittels der Umgebungsluft. Eine zu starke Verschmutzung einer solchen Komponente kann zu einer unzureichenden Zufuhr von Verbrennungsluft und/oder zu einer unzureichenden Kühlung führen. Insbesondere kann die Verschmutzungsursache in der Umgebungsluft enthalten sein. Somit kann die Wechselwirkung mit der Umgebungsluft zu der Verschmutzung führen.

In einer Weiterbildung der Erfindung weist das Garten- und/oder Forstgerät eine Säge, einen Hoch-Entaster, einen Freischneider, ein Blasgerät und/oder einen Laubbläser auf oder ist ein solches spezifisches Garten- und/oder Forstgerät. In der Regel kann ein derart weitergebildetes Garten- und/oder Forstgerät bei seinem Betrieb beziehungsweise seine Komponente der Verschmutzungsursache ausgesetzt sein oder diese sogar selbst verursachen. Insbesondere kann die Säge, der Hoch-Entaster, der Freischneider, das Blasgerät oder der Laubbläser jeweils als handgetragenes Garten- und/oder Forstgerät bezeichnet werden.

In einer Weiterbildung der Erfindung weist die Verschmutzungsursache Staub und/oder Baumharz und/oder Pollen auf oder ist eine solche spezifische Verschmutzungsursache. Der Schmutzparameter weist eine Staubkonzentration und/oder eine Harzmenge und/oder eine Pollenkonzentration auf oder ist ein solcher spezifischer Schmutzparameter. Die Staubkonzentration kann einen Wert und/oder einen Betrag aufweisen oder sein. Die Harzmenge kann einen Wert und/oder einen Betrag aufweisen oder sein. Die Pollenkonzentration kann einen Wert und/oder einen Betrag aufweisen oder sein. Insbesondere kann eine derartige Verschmutzungsursache durch den Betrieb des Garten- und/oder Forstgeräts selbst verursacht sein. Der Staub kann Silikat aufweisen. Die Harzmenge kann von einem Harzanteil in Holz abhängen oder zumindest beeinflusst sein. Der zuvor beschriebene Sensor kann dazu ausgebildet sein, die Staubkonzentration und/oder die Harzmenge und/oder die Pollenkonzentration zu ermitteln und/oder eine Transmission, insbesondere der Umgebungsluft, zu ermitteln, welche von dem Staub und/oder dem Baumharz und/oder dem Pollen beziehungsweise ihrem Umfang oder Ausmaß abhängen oder zumindest beeinflusst sein kann. Der Sensor kann einen Transmissionsermittler aufweisen.

In einer Weiterbildung der Erfindung weist der Schritt a) auf: Empfangen, insbesondere kabelloses oder drahtloses Empfangen, oder Abrufen des Schmutzparameters, insbesondere von einer Datenbank und/oder einem Netzwerk, insbesondere mittels einer Empfangseinheit.

In einer Weiterbildung der Erfindung weist der Schritt a) auf: Ermitteln, insbesondere Erfassen und/oder Empfangen, eines Meteorologieparameters und Ermitteln des Schmutzparameters in Abhängigkeit von oder basierend auf dem ermittelten Meteorologieparameter. Insbesondere kann der Meteorologieparameter eine Lufttemperatur, eine Luftfeuchtigkeit, einen Taupunkt, einen Luftdruck, eine Luftdichte, eine Luftzusammensetzung, eine Windrichtung, eine Windgeschwindigkeit, eine Niederschlagsart, eine Niederschlagsmenge und/oder eine Jahreszeit aufweisen. Von der Jahreszeit können/kann insbesondere die Lufttemperatur und/oder die Luftfeuchtigkeit und/oder der Taupunkt und/oder der Luftdruck und/oder die Luftdichte und/oder die Luftzusammensetzung und/oder die Windrichtung und/oder die Windgeschwindigkeit und/oder die Niederschlagsart und/oder die Niederschlagsmenge abhängen oder zumindest beeinflusst sein. Der Meteorologieparameter kann einen Wert und/oder einen Betrag aufweisen oder sein. Die Verschmutzungsursache beziehungsweise ihr Umfang oder Ausmaß kann von dem Meteorologieparameter abhängen oder zumindest beeinflusst sein. Insbesondere kann Wind die Verschmutzungsursache zu der Komponente hin transportieren oder von dieser wegtransportieren. Zusätzlich oder alternativ kann Niederschlag die Verschmutzungsursache, insbesondere Staub, Baumharz und/oder Pollen, soweit vorhanden entfernen, insbesondere aus der Umgebungsluft auswaschen. Der zuvor beschriebene Sensor kann dazu ausgebildet sein, den Meteorologieparameter zu ermitteln. Der Sensor kann ein Thermometer, ein Hygrometer, ein Taupunktspiegelhygrometer, ein Barometer und/oder einen Niederschlagsmesser aufweisen.

In einer Weiterbildung der Erfindung weist der Schritt a) auf: Ermitteln, insbesondere Erfassen und/oder Empfangen, eines Werkstoffs eines durch das Garten- und/oder Forstgerät zu bearbeitenden Werkstücks oder Objekts und Ermitteln des Schmutzparameters in Abhängigkeit von oder basierend auf dem ermittelten Werkstoff. Insbesondere kann die Verschmutzungsursache beziehungsweise ihr Umfang oder Ausmaß von dem Werkstoff abhängen oder zumindest beeinflusst sein. Das Werkstück kann ein Holzstück aufweisen oder sein, insbesondere ein Baum. Der Werkstoff kann eine Holzart beziehungsweise eine Baumart aufweisen. Von der Holzart beziehungsweise der Baumart kann die Staubkonzentration und/oder die Harzmenge, soweit vorhanden, abhängen oder zumindest beeinflusst sein. Der zuvor beschriebene Sensor kann dazu ausgebildet sein, den Werkstoff zu ermitteln. Der Sensor kann eine Kamera zum Ermitteln eines Bildes des Werkstücks, insbesondere seiner Oberflächenbeschaffenheit, und eine Bildverarbeitungseinrichtung zum Ermitteln des Werkstoffs aus dem Bild aufweisen.

In einer Weiterbildung der Erfindung weist der Schritt a) auf: Ermitteln, insbesondere Erfassen oder Vorgeben, einer Position des Betriebs des Garten- und/oder Forstgeräts und Ermitteln des Schmutzparameters in Abhängigkeit von oder basierend auf der ermittelten Position. Zusätzlich oder alternativ weist der Schritt a) auf: Ermitteln, insbesondere Erfassen oder Vorgeben, einer Kennzeichnung des durch das Garten- und/oder Forstgerät zu bearbeitenden Werkstücks oder Objekts und Ermitteln des Schmutzparameters in Abhängigkeit von oder basierend auf der ermittelten Kennzeichnung. Insbesondere kann aus der ermittelten Position der Schmutzparameter für diese Position und/oder der Meteorologieparameter für diese Position und/oder der Werkstoff für diese Position ermittelt, insbesondere empfangen, werden. Aus der ermittelten Kennzeichnung kann die Position für diese Kennzeichnung und/oder für dieses Werkstück ermittelt werden und somit der Schmutzparameter ermittelt werden, wie zuvor beschrieben. Zusätzlich oder alternativ kann aus der ermittelten Kennzeichnung der Werkstoff des Werkstücks und somit der Schmutzparameter ermittelt werden. Der zuvor beschriebene Sensor kann dazu ausgebildet sein, die Position und/oder die Kennzeichnung zu ermitteln. Der Sensor kann einen Positionsbestimmungssensor aufweisen, insbesondere einen Satelliten-Positionsbestimmungsempfänger. Zusätzlich oder alternativ kann der Sensor ein Lesegerät zum Lesen der Kennzeichnung aufweisen, insbesondere eine Kamera und eine Bildverarbeitungseinrichtung. Insbesondere kann die Kennzeichnung an dem Werkstück angebracht sein, insbesondere von dem Garten- und/oder Forstarbeiter. Die Kennzeichnung kann mindestens ein Zeichen, insbesondere einen Buchstaben und/oder eine Zahl, und/oder einen Code, insbesondere einen Barcode und/oder einen QR-Code, aufweisen. Zusätzlich oder alternativ kann die Kennzeichnung einen NFC-Chip aufweisen oder sein.

In einer Weiterbildung der Erfindung weist die Information den ermittelten Verschmutzungszustand auf oder ist der ermittelte Verschmutzungszustand. Zusätzlich oder alternativ weist das Verfahren den Schritt auf: Ermitteln, insbesondere automatisches Ermitteln, eines Reinigungs- und/oder Wartungshinweises für die Komponente basierend auf dem ermittelten Verschmutzungszustand. Die Information weist den Reinigungs- und/oder Wartungshinweis auf oder ist der Reinigungs- und/oder Wartungshinweis. Dies ermöglicht eine fachgerechte Reinigung und/oder Wartung der Komponente. Insbesondere kann der Reinigungs- und/oder Wartungshinweis darauf hinweisen, dass ein Reinigerspray, das insbesondere einen harzlösenden Stoff aufweisen kann, injiziert werden kann oder sollte.

Des Weiteren bezieht sich die Erfindung auf ein Garten- und/oder Forstgerätesystem, das insbesondere zum Ausführen des zuvor beschriebenen Verfahrens ausgebildet sein kann. Das erfindungsgemäße Garten- und/oder Forstgerätesystem weist eine Schmutzparameter-Ermittlungseinrichtung, eine Verschmutzungszustand-Ermittlungseinrichtung und eine Ausgabe- und/oder Übertragungseinrichtung auf. Die Schmutzparameter-Ermittlungseinrichtung ist zum Ermitteln des mindestens einen Schmutzparameters ausgebildet, der die mindestens eine während des Betriebs des Garten- und/oder Forstgeräts vorherrschende Verschmutzungsursache beschreibt. Die Verschmutzungszustand-Ermittlungseinrichtung ist zum Ermitteln des Verschmutzungszustands der mindestens einen Komponente des Garten- und/oder Forstgeräts basierend auf dem ermittelten Schmutzparameter ausgebildet. Die Ausgabe- und/oder Übertragungseinrichtung ist zum Ausgeben und/oder zum Übertragen der auf dem ermittelten Verschmutzungszustand basierenden Information ausgebildet.

Das Garten- und/oder Forstgerätesystem kann die gleichen Vorteile ermöglichen wie das zuvor beschriebene Verfahren.

Insbesondere kann die Schmutzparameter-Ermittlungseinrichtung eine Empfangseinheit aufweisen. Die Empfangseinheit kann eine UMTS-, WLAN-, und/oder Bluetooth-Empfangseinheit oder eine auf einer anderen Technik basierende Empfangseinheit aufweisen. Zusätzlich oder alternativ kann die Schmutzparameter-Ermittlungseinrichtung den zuvor beschriebenen Sensor aufweisen. Die Verschmutzungszustand-Ermittlungseinrichtung kann eine Rechen- und/oder Speichereinheit aufweisen. Die Ausgabe- und/oder Übertragungseinrichtung kann eine Anzeige, einen Schallerzeuger und/oder eine Vibrationseinrichtung aufweisen. Insbesondere kann die Anzeige dazu ausgebildet sein, die Information in dem Sichtfeld des Garten- und/oder Forstarbeiters anzuzeigen und/oder in dieses zu projizieren oder einzublenden. Die Anzeige kann auf Englisch als Head-up-Display bezeichnet werden. Dies kann auf Englisch als Virtual Reality und/oder als Augmented Reality bezeichnet werden. Insbesondere kann die Anzeige dazu ausgebildet sein, im Bereich oder sogar am Kopf des Garten- und/oder Forstarbeiters angeordnet zu werden. Die Anzeige kann auf Englisch als Head-mounted-Display bezeichnet werden. Zusätzlich oder alternativ kann die Ausgabe- und/oder Übertragungseinrichtung eine Sendeeinheit aufweisen. Die Sendeeinheit kann eine UMTS-, WLAN-, und/oder Bluetooth-Sendeeinheit oder eine auf einer anderen Technik basierende Sendeeinheit aufweisen.

In einer Weiterbildung der Erfindung weist das Garten- und/oder Forstgerätesystem ein mobiles oder portables, insbesondere handgetragenes, Ermittlungsgerät auf. Das mobile Ermittlungsgerät weist die Schmutzparameter-Ermittlungseinrichtung, die Verschmutzungszustand-Ermittlungseinrichtung und/oder die Ausgabe- und/oder Übertragungseinrichtung auf. Das mobile Ermittlungsgerät ermöglicht ein benutzerfreundliches Mitführen. Insbesondere kann das mobile Ermittlungsgerät ein Smartphone und/oder eine Smartwatch aufweisen. Das mobile Ermittlungsgerät kann getrennt von der Komponente, insbesondere von dem Garten- und/oder Forstgerät, ausgebildet sein.

In einer Weiterbildung weist das Garten- und/oder Forstgerätesystem das Garten- und/oder Forstgerät auf.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind. Dabei zeigen:
- Fig. 1: ein Ablaufdiagramm eines erfindungsgemäßen Verfahrens,
- Fig. 2: eine Perspektivansicht eines erfindungsgemäßen Garten- und/oder Forstgerätesystems mit mindestens einem mobilen Ermittlungsgerät und mit einem Garten- und/oder Forstgerät bei einem Bearbeiten eines Werkstücks,
- Fig. 3: eine Längsschnittansicht des Garten- und/oder Forstgeräts der Fig. 2 und
- Fig. 4: eine Vorderansicht des mobilen Ermittlungsgeräts der Fig. 2.

### DETAILLIERTE BESCHREIBUNG DER AUSFÜHRUNGSBEISPIELE

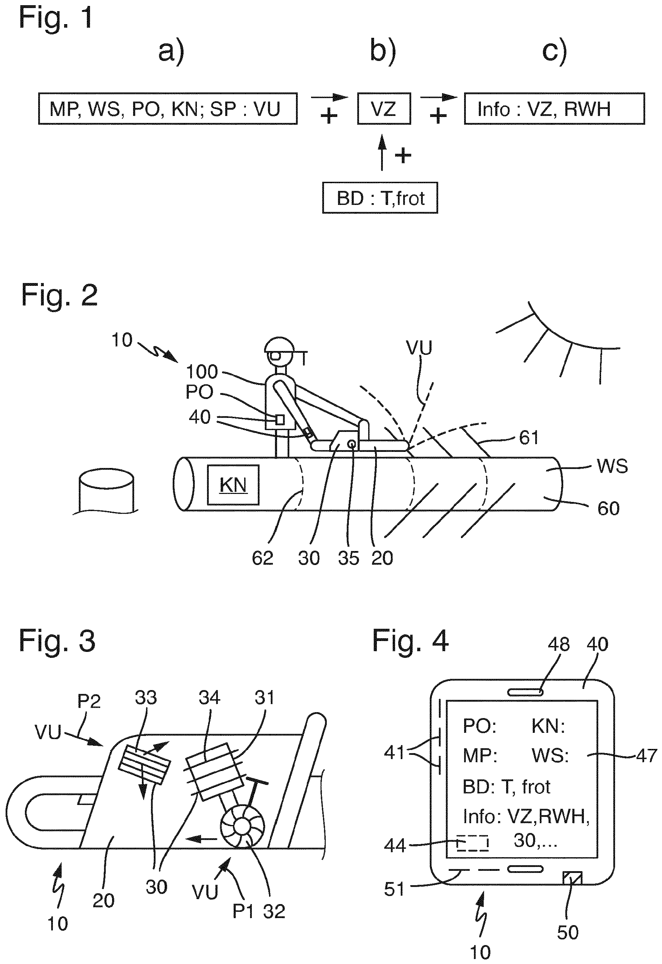

Fig. 2 bis 4 zeigen ein erfindungsgemäßes Garten- und/oder Forstgerätesystem 10. Das Garten- und/oder Forstgerätesystem 10 weist eine Schmutzparameter-Ermittlungseinrichtung 41, 50, 51, eine Verschmutzungszustand-Ermittlungseinrichtung 44 und eine Ausgabe- und/oder Übertragungseinrichtung 41, 47, 48 auf. Die Schmutzparameter-Ermittlungseinrichtung 41, 50, 51 ist zum Ermitteln mindestens eines Schmutzparameters SP beziehungsweise seines Werts ausgebildet. Der Schmutzparameter SP beschreibt mindestens eine während eines Betriebs eines, insbesondere handgetragenen, Garten- und/oder Forstgeräts 20 vorherrschende Verschmutzungsursache VU. Die Verschmutzungszustand-Ermittlungseinrichtung 44 ist zum Ermitteln eines Verschmutzungszustands VZ beziehungsweise seines Werts mindestens einer Komponente 30 des Garten- und/oder Forstgeräts 20 basierend auf dem ermittelten Schmutzparameter SP ausgebildet. Die Ausgabe- und/oder Übertragungseinrichtung 41, 47, 48 ist zum Ausgeben und/oder zum Übertragen einer auf dem ermittelten Verschmutzungszustand VZ basierenden Information Info ausgebildet.

Des Weiteren weist das Garten- und/oder Forstgerätesystem 10 mindestens ein mobiles Ermittlungsgerät 40 auf. Das mobile Ermittlungsgerät 40 weist die Schmutzparameter-Ermittlungseinrichtung 41, 50, 51, die Verschmutzungszustand-Ermittlungseinrichtung 44 und die Ausgabe- und/oder Übertragungseinrichtung 41, 47, 48 auf. In alternativen Ausführungsbeispielen kann es ausreichen, wenn das mobile Ermittlungsgerät entweder die Schmutzparameter-Ermittlungseinrichtung oder die Verschmutzungszustand-Ermittlungseinrichtung oder die Ausgabe- und/oder Übertragungseinrichtung aufweisen kann. Im gezeigten Ausführungsbeispiel weist das Garten- und/oder Forstgerätesystem 10 zwei mobile Ermittlungsgeräte 40 auf. Das eine mobile Ermittlungsgerät 40 weist ein Smartphone auf, das in Fig. 2 in einer Tasche eines Garten- und/oder Forstarbeiters 100 angeordnet ist. Das andere mobile Ermittlungsgerät 40 weist eine Smartwatch auf, die in Fig. 2 am rechten Arm des Garten- und/oder Forstarbeiters 100 angeordnet ist. In alternativen Ausführungsbeispielen kann es ausreichen, wenn das mobile Ermittlungsgerät entweder das Smartphone oder die Smartwatch aufweisen kann.

Außerdem weist das Garten- und/oder Forstgerätesystem 10 das Garten- und/oder Forstgerät 20 auf.

Fig. 1 zeigt ein erfindungsgemäßes Verfahren zum Ermitteln des Verschmutzungszustands VZ der mindestens einen Komponente 30 des Garten- und/oder Forstgeräts 20, insbesondere mittels des zuvor beschriebenen Garten- und/oder Forstgerätesystems 10. Das Verfahren weist die Schritte auf: a) Ermitteln des mindestens einen Schmutzparameters SP, der die mindestens eine während des Betriebs des Garten- und/oder Forstgeräts 20 vorherrschende Verschmutzungsursache VU beschreibt. b) Ermitteln des Verschmutzungszustands VZ der Komponente 30 basierend auf dem ermittelten Schmutzparameter SP. c) Ausgeben und Übertragen der auf dem ermittelten Verschmutzungszustand VZ basierenden Information Info. In alternativen Ausführungsbeispielen kann es ausreichen, wenn der Schritt c) entweder das Ausgeben oder das Übertragen aufweisen kann.

Im Detail weist das Garten- und/oder Forstgerät 20 eine Säge auf, insbesondere eine motorangetriebene Kettensäge. In alternativen Ausführungsbeispielen kann das Garten- und/oder Forstgerät zusätzlich oder alternativ einen Hoch-Entaster, einen Freischneider, ein Blasgerät und/oder einen Laubbläser aufweisen.

In Fig. 2 bearbeitet der Forstarbeiter 100 mit dem Garten- und/oder Forstgerät 20 ein zu bearbeitendes Werkstück 60 in Form eines zuvor mittels des Garten- und/oder Forstgeräts 20 gefällten Baumstamms. Im Detail entfernt der Forstarbeiter 100 Äste 61. Anschließend wird er den Baumstamm 60 mittels des Garten- und/oder Forstgeräts 20 durchtrennen, insbesondere an den durch gestrichelte Linien 62 angedeuteten Stellen. Bei seinem Betrieb war, ist und wird das Garten- und/oder Forstgerät 20 beziehungsweise seine Komponente 30 der Verschmutzungsursache VU ausgesetzt sein, insbesondere diese zumindest in Teilen selbst verursachen.

Im gezeigten Ausführungsbeispiel weist die Verschmutzungsursache VU Staub und Baumharz auf, insbesondere von dem Baumstamm 60 und den Ästen 61. In alternativen Ausführungsbeispielen kann die Verschmutzungsursache entweder Staub oder Baumharz aufweisen. Weiter kann in alternativen Ausführungsbeispielen die Verschmutzungsursache zusätzlich oder alternativ Pollen aufweisen. Im gezeigten Ausführungsbeispiel weist der Schmutzparameter SP eine Staubkonzentration und eine Harzmenge auf. In alternativen Ausführungsbeispielen kann der Schmutzparameter entsprechend entweder eine Staubkonzentration oder eine Harzmenge aufweisen. Weiter kann in alternativen Ausführungsbeispielen der Schmutzparameter zusätzlich oder alternativ eine Pollenkonzentration aufweisen.

Im Detail weist die mindestens eine Komponente 30 einen Zylinder 31, ein Lüfterrad 32, ein Luftfiltergitter 35, einen Luftfilter 33 und eine Kühlrippe 34 auf, wie in Fig. 2 und 3 zu erkennen. Das Luftfiltergitter 35 ist vor dem Lüfterrad 32 angeordnet. In alternativen Ausführungsbeispielen kann es ausreichen, wenn die Komponente 30 entweder den Zylinder oder das Lüfterrad oder das Luftfiltergitter oder den Luftfilter oder die Kühlrippe aufweisen kann. Diese Komponenten 30 wechselwirken mit einer Umgebungsluft des Garten- und/oder Forstgeräts 20, insbesondere zur Verbrennung der Umgebungsluft und/oder zur Kühlung mittels der Umgebungsluft, wie in Fig. 3 durch die Pfeile P1, P2 angedeutet. Eine zu starke Verschmutzung einer solchen Komponente 30 kann zu einer unzureichenden Zufuhr von Verbrennungsluft und/oder zu einer unzureichenden Kühlung führen. Im gezeigten Ausführungsbeispiel ist die Verschmutzungsursache VU in der Umgebungsluft enthalten. Somit führt die Wechselwirkung mit der Umgebungsluft zu der Verschmutzung.

Im Detail weist die Schmutzparameter-Ermittlungseinrichtung mindestens einen Sensor 50, 51 auf, der getrennt von der Komponente 30 ausgebildet ist. Entsprechend weist der Schritt a) auf: Ermitteln des Schmutzparameters SP mittels des mindestens einen Sensors 50, 51.

Im gezeigten Ausführungsbeispiel ist der Sensor 50 als ein Mikrophon ausgebildet. Des Weiteren ist der Sensor 51 als ein Positionsbestimmungssensor ausgebildet, insbesondere als ein Satelliten-Positionsbestimmungsempfänger. Außerdem weist die Schmutzparameter-Ermittlungseinrichtung eine nicht dargestellte Kamera, die an einer Rückseite des mobilen Ermittlungsgeräts 40 angeordnet ist, und eine nicht dargestellte Bildverarbeitungseinrichtung auf. In alternativen Ausführungsbeispielen kann die Schmutzparameter-Ermittlungseinrichtung nur einen Teil der zuvor beschriebenen Sensoren aufweisen. Weiter kann in alternativen Ausführungsbeispielen die Schmutzparameter-Ermittlungseinrichtung zusätzlich oder alternativ andere Sensoren aufweisen.

Entsprechend weist der Schritt a) auf: Ermitteln einer Position PO des Betriebs des Garten- und/oder Forstgeräts 20, insbesondere mittels des Sensors 51, und Ermitteln des Schmutzparameters SP in Abhängigkeit von der ermittelten Position PO.

Des Weiteren weist der Schritt a) auf: Ermitteln einer Kennzeichnung KN des durch das Garten- und/oder Forstgeräts 20 zu bearbeitenden Werkstücks 60, insbesondere mittels der Kamera und der Bildverarbeitungseinrichtung, und Ermitteln des Schmutzparameters SP in Abhängigkeit von der ermittelten Kennzeichnung KN.

Außerdem weist der Schritt a) auf: Ermitteln eines Werkstoffs WS des durch das Garten- und/oder Forstgeräts 20 zu bearbeitenden Werkstücks 60, insbesondere mittels der Kamera und der Bildverarbeitungseinrichtung, und Ermitteln des Schmutzparameters SP in Abhängigkeit von dem ermittelten Werkstoff WS. Im gezeigten Ausführungsbeispiel weist der Werkstoff WS eine Holzart beziehungsweise eine Baumart auf. Von der Holzart beziehungsweise der Baumart hängen die Staubkonzentration und die Harzmenge ab.

Weiter weist die Schmutzparameter-Ermittlungseinrichtung eine Empfangs- und Sendeeinheit 41 auf. Die Empfangs- und Sendeeinheit 41 ist als eine Funk-Antenne ausgebildet.

Entsprechend weist der Schritt a) auf: Empfangen des Schmutzparameters SP, insbesondere von einer Datenbank und/oder einem Netzwerk, insbesondere mittels der Empfangs- und Sendeeinheit 41.

Des Weiteren weist der Schritt a) auf: Ermitteln, insbesondere Empfangen, eines Meteorologieparameters MP, insbesondere mittels der Empfangs- und Sendeeinheit 41, und Ermitteln des Schmutzparameters SP in Abhängigkeit von dem ermittelten Meteorologieparameter MP. Im gezeigten Ausführungsbeispiel weist der Meteorologieparameter MP eine Lufttemperatur, eine Luftfeuchtigkeit, einen Taupunkt, einen Luftdruck, eine Luftdichte, eine Luftzusammensetzung, eine Windrichtung, eine Windgeschwindigkeit, eine Niederschlagsart, eine Niederschlagsmenge und eine Jahreszeit auf. In alternativen Ausführungsbeispielen kann es ausreichen, wenn der Meteorologieparameter nur einen Teil dieser spezifischen Meteorologieparameter aufweisen kann. In der in Fig. 2 gezeigten Situation ist die Jahreszeit Sommer. Die Sonne scheint und die Lufttemperatur ist relativ hoch. Es gibt keinen Niederschlag, welcher insbesondere die Verschmutzungsursache VU in Form des Staubs und des Baumharzes aus der Umgebungsluft auswaschen könnte. Somit ist der Schmutzparameter SP beziehungsweise sein Wert, insbesondere seine Staubkonzentration und seine Harzmenge, relativ hoch. Dies kann zeitlich relativ schnell zu einem relativ starken Verschmutzungszustand VZ der Komponente 30 führen. In der Regel wird in einer anderen Jahreszeit, beispielsweise im Winter, bei einer relativ niedrigeren Lufttemperatur oder bei Niederschlag in Form von Regen der Schmutzparameter beziehungsweise sein Wert relativ niedriger sein. In der Regel wird dies zeitlich relativ langsam zu einem relativ starken Verschmutzungszustand der Komponente führen.

Im Detail kann aus der ermittelten Position PO der Schmutzparameter SP für diese Position PO und/oder der Meteorologieparameter MP für diese Position PO und/oder der Werkstoff WS für diese Position PO ermittelt, insbesondere empfangen, werden. Des Weiteren kann aus der ermittelten Kennzeichnung KN die Position PO für diese Kennzeichnung KN und/oder für das zu bearbeitende Werkstück 60 ermittelt werden und somit der Schmutzparameter SP ermittelt werden, wie zuvor beschrieben. Zusätzlich oder alternativ kann aus der ermittelten Kennzeichnung KN der Werkstoff WS des zu bearbeitenden Werkstücks 60 und somit der Schmutzparameter SP ermittelt werden.

In alternativen Ausführungsbeispielen kann es ausreichen, wenn der Schritt a) aufweisen kann: entweder unmittelbar Ermitteln oder Empfangen des Schmutzparameters oder Ermitteln des Meteorologieparameters oder Ermitteln des Werkstoffs oder Ermitteln der Position oder Ermitteln der Kennzeichnung.

Im gezeigten Ausführungsbeispiel ist der Verschmutzungszustand VZ der Komponente 30 von dem Betrieb des Garten- und/oder Forstgeräts 20 abhängig. Die Komponente 30 ist während des Betriebs des Garten- und/oder Forstgeräts 20 der Verschmutzungsursache VU ausgesetzt. Das Verfahren weist den Schritt auf: Ermitteln von Daten des Betriebs BD des Garten- und/oder Forstgeräts 20. Der Schritt b) weist auf: Ermitteln des Verschmutzungszustands VZ der Komponente 30 basierend auf den ermittelten Daten des Betriebs BD.

Im Detail weisen die Daten des Betriebs BD eine Laufzeit T des Garten- und/oder Forstgeräts 20 auf, insbesondere einen Drehzahlverlauf frot des Garten- und/oder Forstgeräts 20. Im gezeigten Ausführungsbeispiel wird die Laufzeit T und der Drehzahlverlauf frot durch Erfassen eines von dem Betrieb des Garten -und/oder Forstgeräts 20 erzeugten Schalls ermittelt, insbesondere mittels des zuvor beschriebenen Sensors 50, der als ein Mikrophon ausgebildet ist.

In der in Fig. 2 gezeigten Situation des relativ hohe Werts des Schmutzparameters SP wird eine relativ lange Laufzeit T, und insbesondere eine relativ hohe Drehzahl frot, des Garten- und/oder Forstgeräts 20 zu einem relativ starken Verschmutzungszustand VZ der Komponente 30 führen. Eine relativ kurze Laufzeit T, und insbesondere eine relativ niedrige Drehzahl frot, des Garten- und/oder Forstgeräts 20 wird zu einem relativ niedrigeren Verschmutzungszustand VZ der Komponente 30 führen. In einer anderen Situation eines relativ niedrigen Werts des Schmutzparameters wird ein relativ lange Laufzeit, und insbesondere eine relativ hohe Drehzahl, des Garten- und/oder Forstgeräts ebenfalls zu einem relativ niedrigeren Verschmutzungszustand VZ der Komponente führen. Eine relativ kurze Laufzeit, und insbesondere eine relativ niedrige Drehzahl, des Garten- und/oder Forstgeräts wird kaum oder nicht zu einer Verschmutzung der Komponente und somit zu einem Verschmutzungszustand gleich Null führen.

Im Detail weist die Verschmutzungszustand-Ermittlungseinrichtung 44 eine Rechen- und/oder Speichereinheit auf. Diese ermittelt basierend auf dem Schmutzparameter SP beziehungsweise seinem Wert und basierend auf den ermittelten Daten des Betriebs BD den Verschmutzungszustand VZ der Komponente 30.

Des Weiteren weist die Ausgabe- und/oder Übertragungseinrichtung eine Anzeige 47 in Form eines Touchscreens, einen Schallerzeuger 48 in Form eines Lautsprechers und eine nicht dargestellte Vibrationseinrichtung auf. In alternativen Ausführungsbeispielen kann es ausreichen, wenn die Ausgabe- und/oder Übertragungseinrichtung entweder die Anzeige oder den Schallerzeuger oder die Vibrationseinrichtung aufweisen kann.

Entsprechend wird die Information Info optisch, akustisch und/oder haptisch ausgegeben, insbesondere rechtzeitig vor einem zu starken Verschmutzungszustand VZ der Komponente 30.

Außerdem weist die Ausgabe- und/oder Übertragungseinrichtung die Empfangs- und Sendeeinheit 41 auf.

Entsprechend kann die Information Info an eine Datenbank und/oder ein Netzwerk übertragen werden.

Im Detail weist die Information Info den ermittelten Verschmutzungszustand VZ auf, insbesondere für jede Komponente 30, 31, 32, 33, 34, 35 aufgeschlüsselt.

Des Weiteren weist das Verfahren den Schritt auf: Ermitteln eines Reinigungs- und/oder Wartungshinweises RWH für die Komponente 30 basierend auf dem ermittelten Verschmutzungszustand VZ. Die Information Info weist den Reinigungs- und/oder Wartungshinweis RWH auf, insbesondere für jede Komponente 30 aufgeschlüsselt.

Wie die gezeigten und oben erläuterten Ausführungsbeispiele deutlich machen, stellt die Erfindung ein vorteilhaftes Verfahren zum Ermitteln eines Verschmutzungszustands mindestens einer Komponente eines Garten- und/oder Forstgeräts bereit, das verbesserte Eigenschaften aufweist, insbesondere mehr Funktionalitäten, sowie ein Garten- und/oder Forstgerätesystem. Insbesondere ermöglichen das Verfahren und das Garten- und/oder Forstgerätesystem ein mittelbares Ermitteln des Verschmutzungszustands der Komponente. Des Weiteren ermöglichen das Verfahren und das Garten- und/oder Forstgerätesystem aufgrund einer auf dem ermittelten Verschmutzungszustand basierenden Information sich um die Komponente zu kümmern oder Vorkehrungen für diese zu treffen, insbesondere bevor diese zu stark verschmutzt sein kann.

## Patentansprüche

1. Verfahren zum Ermitteln eines Verschmutzungszustands (VZ) mindestens einer Komponente (30) eines Garten- und/oder Forstgeräts (20), wobei das Verfahren die Schritte aufweist:
a) Ermitteln mindestens eines Schmutzparameters (SP), der mindestens eine während eines Betriebs des Garten- und/oder Forstgeräts (20) vorherrschende Verschmutzungsursache (VU) beschreibt,
b) Ermitteln des Verschmutzungszustands (VZ) der Komponente (30) basierend auf dem ermittelten Schmutzparameter und
c) Ausgeben und/oder Übertragen einer auf dem ermittelten Verschmutzungszustand basierenden Information (Info).

2. Verfahren nach Anspruch 1,
- wobei der Schritt a) aufweist: Ermitteln des Schmutzparameters (SP) mittels mindestens eines Sensors (50), der getrennt von der Komponente (30) ausgebildet ist.

3. Verfahren nach einem der vorhergehenden Ansprüche,
- wobei der Verschmutzungszustand (VZ) der Komponente (30) von dem Betrieb des Garten- und/oder Forstgeräts (20) abhängig ist, wobei die Komponente während des Betriebs des Garten- und/oder Forstgeräts der Verschmutzungsursache ausgesetzt ist,
- wobei das Verfahren den Schritt aufweist: Ermitteln von Daten des Betriebs (BD) des Garten- und/oder Forstgeräts (20) und
- wobei der Schritt b) aufweist: Ermitteln des Verschmutzungszustands (VZ) der Komponente (30) basierend auf den ermittelten Daten des Betriebs (BD).

4. Verfahren nach Anspruch 3,
- wobei die Daten des Betriebs (BD) eine Laufzeit (T) des Garten- und/oder Forstgeräts (20) aufweisen, insbesondere einen Drehzahlverlauf (frot) des Garten- und/oder Forstgeräts.

5. Verfahren nach einem der vorhergehenden Ansprüche,
- wobei die Komponente (30) einen Zylinder (31), ein Lüfterrad (32), ein Luftfiltergitter (35), einen Luftfilter (33) und/oder eine Kühlrippe (34) aufweist.

6. Verfahren nach einem der vorhergehenden Ansprüche,
- wobei das Garten- und/oder Forstgerät (20) eine Säge, einen Hoch-Entaster, einen Freischneider, ein Blasgerät und/oder einen Laubbläser aufweist.

7. Verfahren nach einem der vorhergehenden Ansprüche,
- wobei die Verschmutzungsursache (VU) Staub und/oder Baumharz und/oder Pollen aufweist und wobei der Schmutzparameter (SP) eine Staubkonzentration und/oder eine Harzmenge und/oder eine Pollenkonzentration aufweist.

8. Verfahren nach einem der vorhergehenden Ansprüche,
- wobei der Schritt a) aufweist: Empfangen des Schmutzparameters (SP).

9. Verfahren nach einem der vorhergehenden Ansprüche,
- wobei der Schritt a) aufweist: Ermitteln eines Meteorologieparameters (MP) und Ermitteln des Schmutzparameters (SP) in Abhängigkeit von dem ermittelten Meteorologieparameter.

10. Verfahren nach einem der vorhergehenden Ansprüche,
- wobei der Schritt a) aufweist: Ermitteln eines Werkstoffs (WS) eines durch das Garten- und/oder Forstgerät (20) zu bearbeitenden Werkstücks (60) und Ermitteln des Schmutzparameters (SP) in Abhängigkeit von dem ermittelten Werkstoff.

11. Verfahren nach einem der vorhergehenden Ansprüche,
- wobei der Schritt a) aufweist: Ermitteln einer Position (PO) des Betriebs des Garten- und/oder Forstgeräts (20) und Ermitteln des Schmutzparameters (SP) in Abhängigkeit von der ermittelten Position und/oder
- wobei der Schritt a) aufweist: Ermitteln einer Kennzeichnung (KN) eines durch das Garten- und/oder Forstgerät (20) zu bearbeitenden Werkstücks (60) und Ermitteln des Schmutzparameters (SP) in Abhängigkeit von der ermittelten Kennzeichnung.

12. Verfahren nach einem der vorhergehenden Ansprüche,
- wobei die Information (Info) den ermittelten Verschmutzungszustand (VZ) aufweist und/oder
- wobei das Verfahren den Schritt aufweist: Ermitteln eines Reinigungs- und/oder Wartungshinweises (RWH) für die Komponente (30) basierend auf dem ermittelten Verschmutzungszustand und wobei die Information den Reinigungs- und/oder Wartungshinweis aufweist.

13. Garten- und/oder Forstgerätesystem (10), aufweisend:
- eine Schmutzparameter-Ermittlungseinrichtung (41, 50, 51), die zum Ermitteln mindestens eines Schmutzparameters (SP) ausgebildet ist, der mindestens eine während eines Betriebs eines Garten- und/oder Forstgeräts (20) vorherrschende Verschmutzungsursache (VU) beschreibt,
- eine Verschmutzungszustand-Ermittlungseinrichtung (44), die zum Ermitteln eines Verschmutzungszustands (VZ) mindestens einer Komponente (30) des Garten- und/oder Forstgeräts (20) basierend auf dem ermittelten Schmutzparameter ausgebildet ist, und
- eine Ausgabe- und/oder Übertragungseinrichtung (41, 47, 48), die zum Ausgeben und/oder zum Übertragen einer auf dem ermittelten Verschmutzungszustand basierenden Information (Info) ausgebildet ist.

14. Garten- und/oder Forstgerätesystem (10) nach Anspruch 13, aufweisend:
- ein mobiles Ermittlungsgerät (40), das die Schmutzparameter-Ermittlungseinrichtung (41, 50, 51), die Verschmutzungszustand-Ermittlungseinrichtung (44) und/oder die Ausgabe- und/oder Übertragungseinrichtung (41, 47, 48) aufweist.

15. Garten- und/oder Forstgerätesystem (10) nach Anspruch 13 oder 14, aufweisend:
- das Garten- und/oder Forstgerät (20).
